# EUROPEAN PATENT SPECIFICATION

(11) **EP 3 081 168 B1**
(45) Date of publication and mention of the grant of the patent: **26.02.2020**
(21) Application number: 14869387.2
(22) Date of filing: 10.12.2014
(51) Int. Cl.: A61B 8/12, A61B 1/00, A61B 10/04, A61B 17/00, A61B 17/34, A61M 25/00, A61M 31/00

(54) **PUNCTURE NEEDLE FOR ULTRASONIC ENDOSCOPE**
PUNKTIONSNADEL FÜR ULTRASCHALLENDOSKOP
AIGUILLE DE PONCTION POUR ENDOSCOPE ULTRASONORE

(30) Priority: 12.12.2013 JP 2013257469
(43) Date of publication of application: 19.10.2016
(73) Proprietor: Olympus Corporation, Tokyo 192-8507 (JP)
(72) Inventor: SATO, Masatoshi, Hachioji-shi, Tokyo 192-8507 (JP)
(74) Representative: Gunzelmann, Rainer
(86) International application number: PCT/JP2014/082671
(87) International publication number: WO 2015/087910

(56) References cited:
- WO-A1-2007/037326
- DE-A1-102005 012 574
- JP-A- 2002 306 497
- JP-A- 2012 515 603
- US-A- 5 221 269
- US-A1- 2005 267 495
- US-A1- 2010 010 293
- US-A1- 2012 172 896
- US-A1- 2013 172 758
- US-A1- 2013 325 038
- US-B1- 6 419 621

## Description

### Technical Field

The present invention relates to a puncture needle for an ultrasound endoscope which is inserted into a body cavity and used for delivering a medicine or a treatment device into a body.

Priority is claimed on Japanese Patent Application No. 2013-257469, filed December 12, 2013.

### Background Art

Conventionally, a surgical technique for aspirating and collecting a tissue in a body cavity or a body fluid has been performed for examining or diagnosing an affected area in the body cavity. The surgical technique is performed by, while observing the inside of the body cavity by means of an ultrasound endoscope, piercing a digestive tract wall or the like of a stomach or a duodenum using a puncture needle, and puncturing, with the puncture needle, a target site of an underlying organ such as a pancreas, a liver, and a kidney. The surgical technique is called an endoscopic ultrasound-guided fine needle aspiration (EUS-FNA).

In recent years, as an advanced application of the EUS-FNA surgical technique, a surgical technique for treatment has been studied which delivers a substance such as a medicine, a marker, and a radiation source directly to an interest site from a puncture needle, instead of aspirating a tissue or a body fluid. By using this surgical technique for treatment, a treatment effect is expected to be improved and a side effect is expected to be reduced since a substance is accurately delivered to an interest site. Therefore, it is preferable to perform the surgical technique while observing, through an ultrasound endoscope, a substance that is actually being delivered.

In addition, an apparatus that places a tool in a living tissue is known (for example, refer to Patent Literature 1 and Patent Literature 2). Specifically, a tool such as a clip and a fastener to be placed in a living tissue is loaded into a distal end of a puncture needle, and placed by the apparatus.

US 2012/172896 A1 discloses a puncture needle configured to be inserted in an ultrasound endoscope, the puncture needle comprising a needle tube, a wire having a proximal end and a distal end, the wire having elasticity such that the wire is restored to a wind shape in an absence of external force, the wire being stretched and loaded into the internal space of the needle tube against restoration to the wind shape such that the wire is supported by portions of the inner surface of the needle tube which are spaced apart from one another in a direction of the longitudinal axis of the needle tube;a slit part formed to open from the inner surface to the outer surface of the needle tube, the slit part having a pair of wall surfaces which are spaced apart from one another, the slit part having an opening width larger than an external diameter of the wire; and a stylet configured to push the proximal end of the wire loaded within the needle tube, wherein the needle tube has a needle tube distal part having a needle tip formed to be sharpened to puncture a tissue within a body cavity.

### Citation List

### Patent Literature

Patent Literature 1: Published Japanese Translation No. 2000-515054 of the PCT International Publication
Patent Literature 2: Published Japanese Translation No. 2008-504943 of the PCT International Publication

### Summary of Invention

### Technical Problem

In a case where a tool to be placed in a living tissue is released from a puncture needle toward the living tissue, it is preferable, in terms of simplicity of operation and safety, that the released tool can be observed through an endoscope. In a case where an ultrasound endoscope is used, however, there is a problem that a position of a tool cannot be recognized once the tool is away from an ultrasound scanning plane defined by the ultrasound endoscope.

The present invention has been made in consideration of the above-mentioned circumstances, and an object thereof is to provide a puncture needle for an ultrasound endoscope that allows an ultrasound endoscope to recognize a position of a tool released from the puncture needle. Solution to Problem

A puncture needle according to the present invention includes the features of claim 1. Preferred embodiments of the invention are defined by the dependent claims.

According to a first aspect of the present disclosure, a puncture needle for an ultrasound endoscope includes: a sheath configured to be freely advanced or retracted in an insertion channel of an ultrasound endoscope, the sheath being configured to be capable of being inserted into the insertion channel; a needle tube having elasticity, the needle tube being formed to be sharpened in a distal direction to puncture a tissue within a body cavity, the needle tube having a curved shape part at least in a vicinity of a distal part in an absence of external force, the needle tube being capable of being inserted into the sheath; a wire having elasticity, the wire being formed in a wind shape or a coil shape in an absence of external force, the wire being capable of being loaded into the needle tube in a straight state; and a releasing mechanism which is disposed at a proximal part of the needle tube and pushes, in the distal direction of the needle tube, the wire loaded within the needle tube to release the wire from the needle tube to an outside. The needle tube has a side hole configured in such a manner that, among a tube wall of the needle tube at a side more distal than the curved shape part, a part of a tube wall is cut off, the tube wall crossing a plane including a central line of the curved shape part, the tube wall being positioned on an inside of a curve of the curved shape part, and the wire being capable of being delivered from the side hole. At least a part of the wire is positioned at the curved shape part in a state that the wire is stretched and loaded into the needle tube.

According to a second aspect of the present disclosure, in the puncture needle according to the first aspect, the needle tube may have a distal end opening inclined so as to have a distal end and a proximal end on a plane that is the same as a plane including a longitudinal central axis at the curved shape part. The proximal end of the distal end opening may be in communication with a distal end of the side hole.

According to a third aspect of the present disclosure, in the puncture needle according to the first or second aspect, among both ends of the wire, an end positioned at a distal side of the needle tube may be positioned at the curved shape part in the state that the wire is stretched and loaded into the needle tube.

According to a fourth aspect of the present disclosure, in the puncture needle according to any one of the first to third aspects, a central part of the wire may be positioned at the curved shape part in the state that the wire is stretched and loaded into the needle tube.

According to a fifth aspect of the present disclosure, in the puncture needle according to any one of the first to fourth aspects, a proximal end of the slit part may be positioned at the curved shape part.

### Advantageous Effects of Invention

According to the above-mentioned aspects, a position of a tool released from the puncture needle can be recognized by the ultrasound endoscope.

### Brief Description of Drawings

Fig. 1 is an overall view showing an ultrasound endoscope which is used in combination with a puncture needle for an ultrasound endoscope according to a first embodiment of the present disclosure.
Fig. 2 is a perspective view showing a distal end part of the ultrasound endoscope.
Fig. 3 is a front view of the distal end part of the ultrasound endoscope.
Fig. 4 is a perspective cross-sectional view of the distal end part of the ultrasound endoscope.
Fig. 5 is an overall view showing another ultrasound endoscope which is used in combination with the puncture needle for an ultrasound endoscope according to the first embodiment of the present disclosure.
Fig. 6 is a perspective view showing a distal end part of another ultrasound endoscope according to the first embodiment of the present disclosure.
Fig. 7 is a front view showing the distal end part of the other ultrasound endoscope.
Fig. 8 is a perspective cross-sectional view of the distal end part of the other ultrasound endoscope.
Fig. 9 is an overall external view of the puncture needle for an ultrasound endoscope according to the first embodiment of the present invention.
Fig. 10 is an overall cross-sectional view of the puncture needle for an ultrasound endoscope according to the first embodiment of the present invention.
Fig. 11 is an overall cross-sectional view of the puncture needle for an ultrasound endoscope according to the first embodiment of the present invention.
Fig. 12A is a view showing a distal end side of a needle tube of the puncture needle for an ultrasound endoscope according to the first embodiment of the present invention.
Fig. 12B is a view showing the distal end side of the needle tube of the puncture needle for an ultrasound endoscope according to the first embodiment of the present invention.
Fig. 13A is a view showing a distal end side of another exemplary needle tube according to the first embodiment of the present invention.
Fig. 13B is a view showing a distal end side of another exemplary needle tube according to the first embodiment of the present invention.
Fig. 14A is a view of an implant to be housed within the needle tube according to the first embodiment of the present invention and a second embodiment not forming part of the present invention.
Fig. 14B is a view showing the implant to be housed within the needle tube according to the first embodiment of the present invention, housed in the needle tube according to the first embodiment.
Fig. 15 is a view showing operation of the ultrasound endoscope during the use of the puncture needle for an ultrasound endoscope according to the first embodiment of the present invention.
Fig. 16A is a view showing operation of the puncture needle for an ultrasound endoscope according to the first embodiment within the bent ultrasound endoscope.
Fig. 16B is a view showing the operation of the puncture needle for an ultrasound endoscope according to the first embodiment within the bent ultrasound endoscope.
Fig. 16C is a view showing the operation of the puncture needle for an ultrasound endoscope according to the first embodiment within the bent ultrasound endoscope.
Fig. 17 is a view showing the implant discharged from the puncture needle for an ultrasound endoscope according to the first embodiment.
Fig. 18 is a view showing the puncture needle for an ultrasound endoscope according to the first embodiment inserted into the other ultrasound endoscope.
Fig. 19 is a view showing a distal end side of a needle tube of a puncture needle for an ultrasound endoscope according to a second embodiment not forming part of the present invention.
Fig. 20 is a view showing the distal end side of the needle tube of the puncture needle for an ultrasound endoscope according to the second embodiment not forming part of the present invention.
Fig. 21 is a view showing the distal end side of the needle tube of the puncture needle for an ultrasound endoscope according to the second embodiment not forming part of the present invention.
Fig. 22 is a partial cross-sectional view showing an exemplary design change for the above-mentioned embodiments.
Fig. 23 is a partial cross-sectional view showing another exemplary design change for the above-mentioned embodiments.
Fig. 24 is a partial cross-sectional view showing still another exemplary design change for the above-mentioned embodiments.

### Description of Embodiments

Hereinafter, embodiments of the present invention and of the present disclosure will be described with reference to the drawings.

### (First Embodiment)

A puncture needle for an ultrasound endoscope according to the present embodiment is used in combination with an ultrasound endoscope.

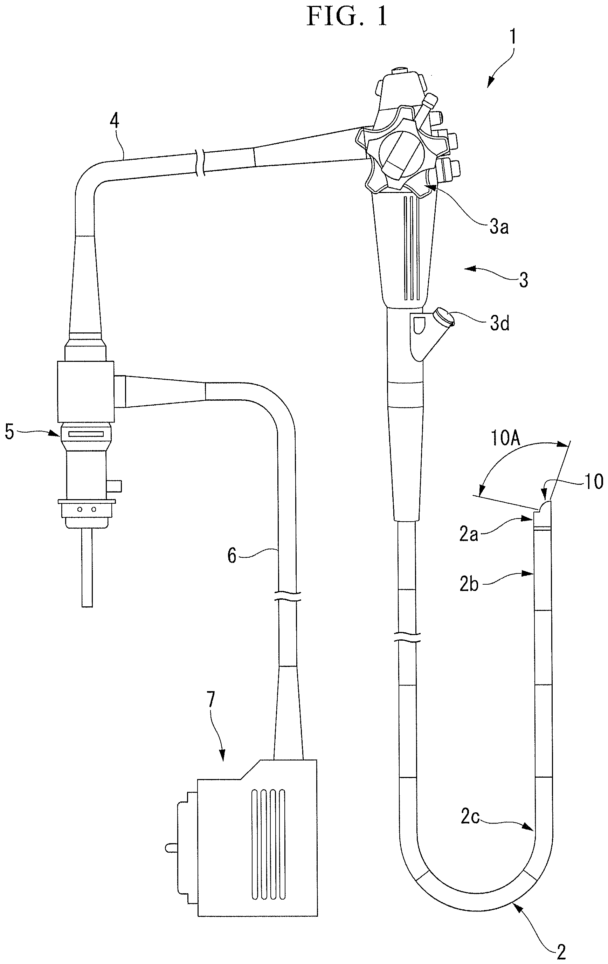

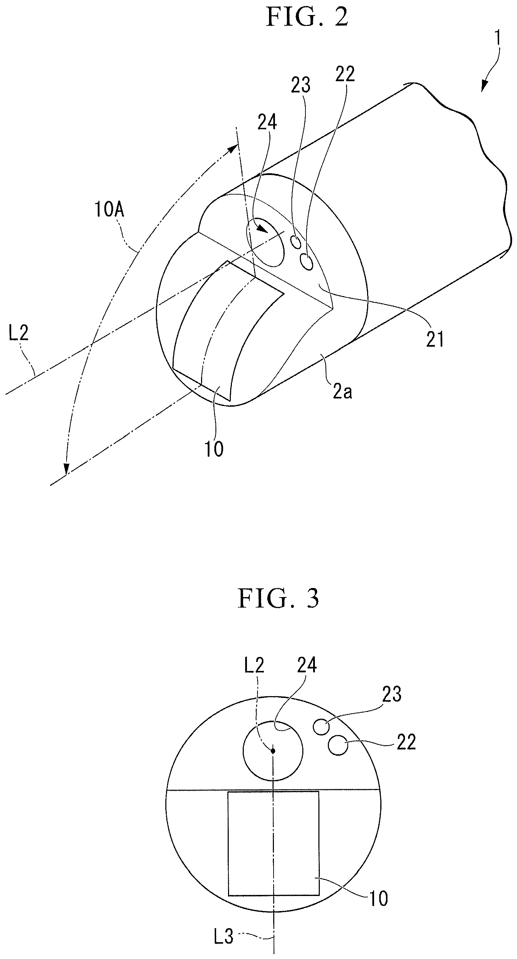

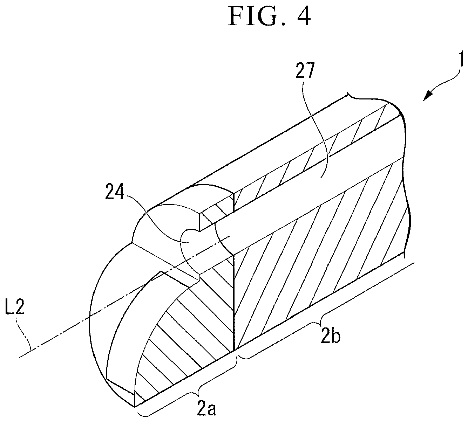

The ultrasound endoscope according to the present embodiment will be described using Figs. 1 to 4. Fig. 1 is a view showing a configuration of the ultrasound endoscope. Fig. 2 is a perspective view showing a distal end part of the ultrasound endoscope. Fig. 3 is a front view of the distal end part shown in Fig. 2 viewed from the front. Fig. 4 is a perspective cross-sectional view of the distal end part of the ultrasound endoscope.

The ultrasound endoscope 1 includes a thin elongated insertion part 2 inserted into a body cavity, an operation part 3 provided at a proximal end of the insertion part 2, and a universal cord 4 extending from a side part of the operation part 3.

A proximal end part of the universal cord 4 is provided with an endoscope connector 5. An ultrasound cable 6 extends from a side part of the endoscope connector 5. A proximal end part of the ultrasound cable 6 is provided with an ultrasound connector 7.

In order from a distal end side of the ultrasound endoscope 1, the insertion part 2 includes a hard part 2a formed of a hard member, a bendable part 2b configured to be bent, and a flexible tube part 2c which is long and extends from a proximal end of the bendable part 2b to a distal end of the operation part 3 and has flexibility, wherein the hard part 2a, the bendable part 2b, and the flexible tube part 2c are connected to one another.

An ultrasound transducer part 10 forms an ultrasound observation plane 10A that scans a forward direction with respect to an insertion axis direction. In other words, the ultrasound transducer part 10 has the ultrasound observation plane 10A that scans the forward direction. A signal cable (not shown) is connected to the ultrasound transducer part 10. The signal cable passes through the insertion part 2, the operation part 3, the universal cord 4, the endoscope connector 5, and the ultrasound cable 6 and extends to the ultrasound connector 7.

The ultrasound connector 7 is connected to an ultrasound observation apparatus (not shown). The ultrasound observation apparatus exchanges a signal with an ultrasound transducer through the signal cable, and converts a signal received from the ultrasound transducer to an ultrasound image to display the ultrasound image on a monitor (not shown).

The operation part 3 is provided with an angle knob 3a for performing a curving operation. When an operator appropriately operates the angle knob 3a, a curve wire (not shown) operated in accordance with the operation is pulled and loosened, whereby the bendable part 2b is operated to be bent.

As shown in Fig. 2, the ultrasound transducer part 10 is configured to project from a distal end surface 21 of the hard part 2a. Furthermore, the distal end surface 21 of the hard part 2a is provided with an observing window 22, an illumination window 23, and an insertion channel outlet 24. The observing window 22 constitutes the farthest distal end side of an observing optical system (not shown). The illumination window 23 constitutes the farthest distal end side of an illumination optical system (not shown). The insertion channel outlet 24 is an opening of a treatment tool insertion channel from which a treatment tool such as a puncture needle is guided. The insertion channel outlet 24 is provided substantially in parallel with a longitudinal axis direction of the hard part 2a, and connected to the treatment tool insertion channel (hereinafter abbreviated as "insertion channel") 27 arranged within the insertion part 2 (refer to Fig. 4).

The observing optical system and the illumination optical system (not shown) extend through the insertion part 2, the operation part 3, and the universal cord 4 to the endoscope connector 5. The endoscope connector 5 is connected to an endoscope observation apparatus (not shown). The endoscope observation apparatus transmits illumination light through the illumination optical system to the illumination window 23. The illumination light illuminates the front of the hard part 2a. The endoscope observation apparatus converts, to an observing image, a signal delivered from the observing window 22 through the observing optical system, and displays the observing image on a monitor (not shown). Therefore, an observing image illuminated with illumination light is displayed on the monitor.

A proximal end side of the insertion channel 27 is in communication with a treatment tool insertion port 3d provided in the operation part 3. A proximal end part of the treatment tool insertion port 3d is formed in a luer-lock shape capable of connecting a syringe. A treatment tool inserted through the treatment tool insertion port 3d is guided from the insertion channel outlet 24.

A central axis L2 of the insertion channel outlet 24 is substantially in parallel with the longitudinal axis direction of the hard part 2a. A surface defined by the central axis L2 and a central line L3 in a vertical direction of the ultrasound transducer part 10 is configured to substantially coincide with the ultrasound observation plane 10A. Since the treatment tool guided from the insertion channel outlet 24 is guided onto the ultrasound observation plane 10A, the treatment tool is displayed visibly on an ultrasound image.

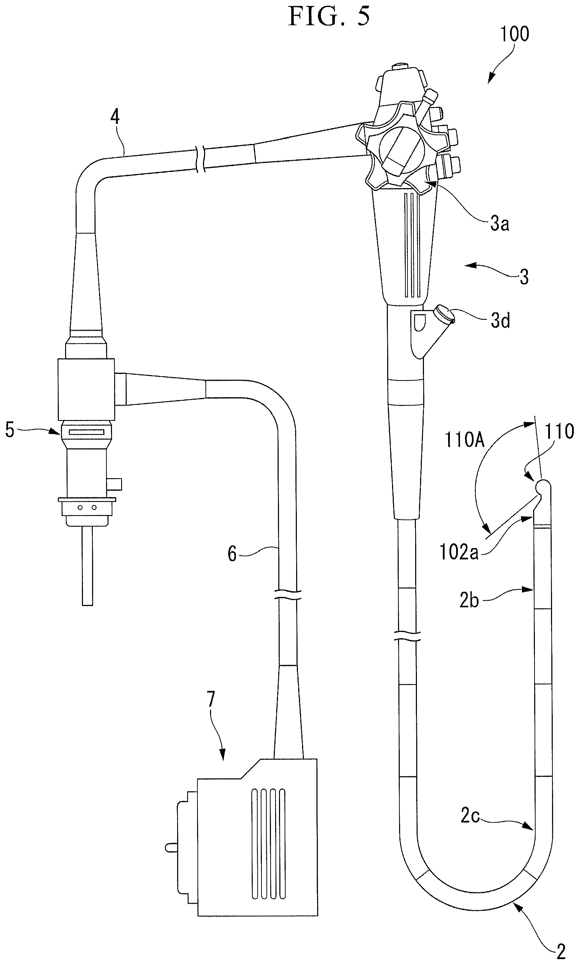

Another ultrasound endoscope which can be used in the present embodiment will be described using Figs. 5 to 8.

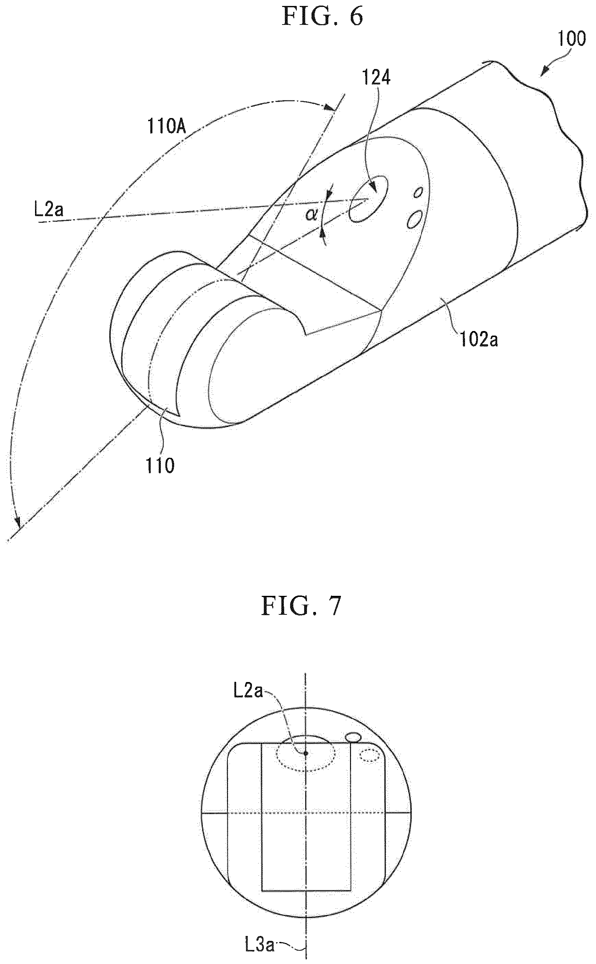

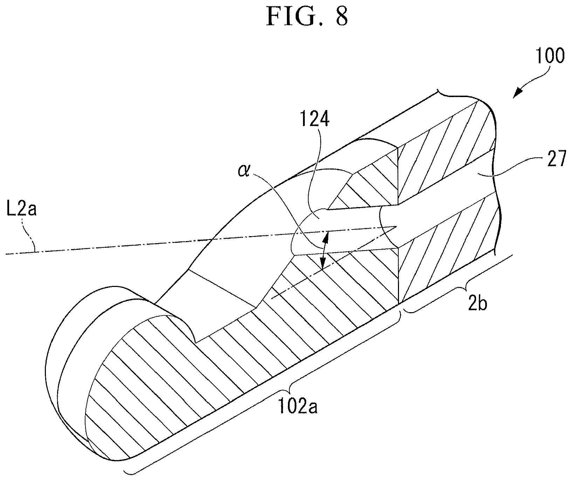

Fig. 5 is a view showing a configuration of the ultrasound endoscope 100. Fig. 6 is a perspective view showing a distal end part of the ultrasound endoscope 100. Fig. 7 is a front view of the distal end part shown in Fig. 5 viewed from the front. Fig. 8 is a perspective cross-sectional view of the distal end part of the ultrasound endoscope 100.

In the ultrasound endoscope 100, components having the same configurations as those of the ultrasound endoscope 1 which has already been described are denoted by the same reference signs in Figs. 5 to 8. A difference from the ultrasound endoscope 1 is that an ultrasound transducer part 110 at the distal end is larger than the ultrasound transducer part 10 according to the first embodiment. As a result, an ultrasound observation plane 110A that scans a forward direction with respect to an insertion axis direction is formed at a wider angle.

Furthermore, as shown in Fig. 6, an insertion channel outlet 124 of the ultrasound endoscope 100 is provided so as to be inclined by an angle α with respect to a longitudinal axis direction of a hard part 102a such that a treatment tool guided from the insertion channel outlet does not come into contact with the ultrasound transducer part 110 formed in a large size.

However, a surface configured by a central axis L2a in a longitudinal direction of the insertion channel outlet 124 and a central line L3a in a vertical direction of the ultrasound transducer part 110 is configured to substantially coincide with the ultrasound observation plane 110A. This configuration is the same as that of the ultrasound endoscope 1. Therefore, the treatment tool guided from the insertion channel outlet 124 is guided onto the ultrasound observation plane 110A, and displayed visibly on an ultrasound image.

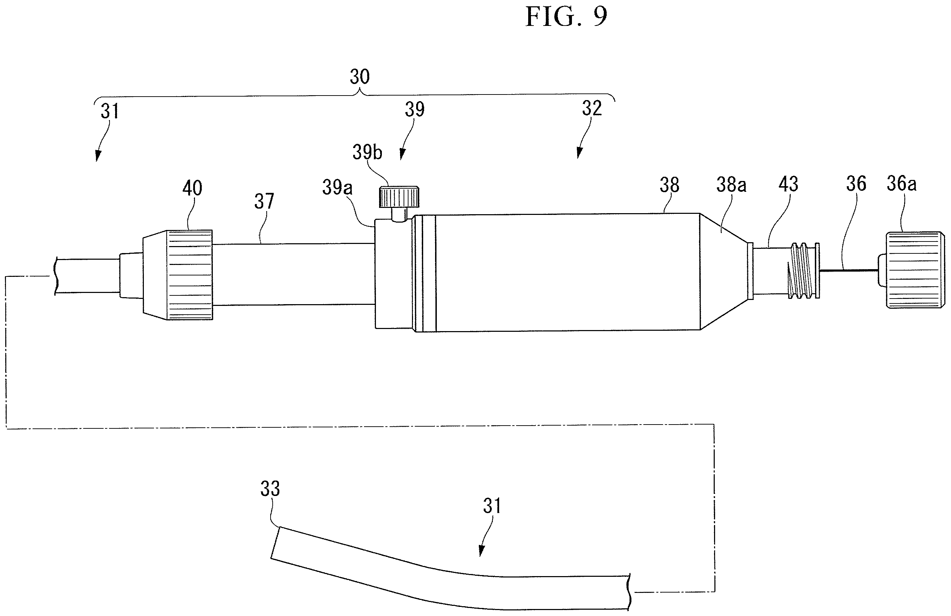

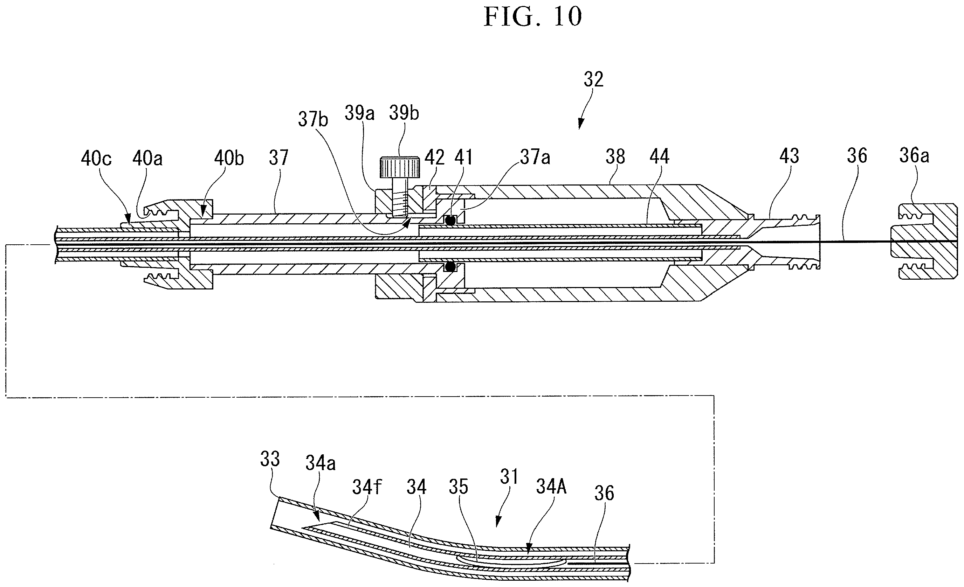

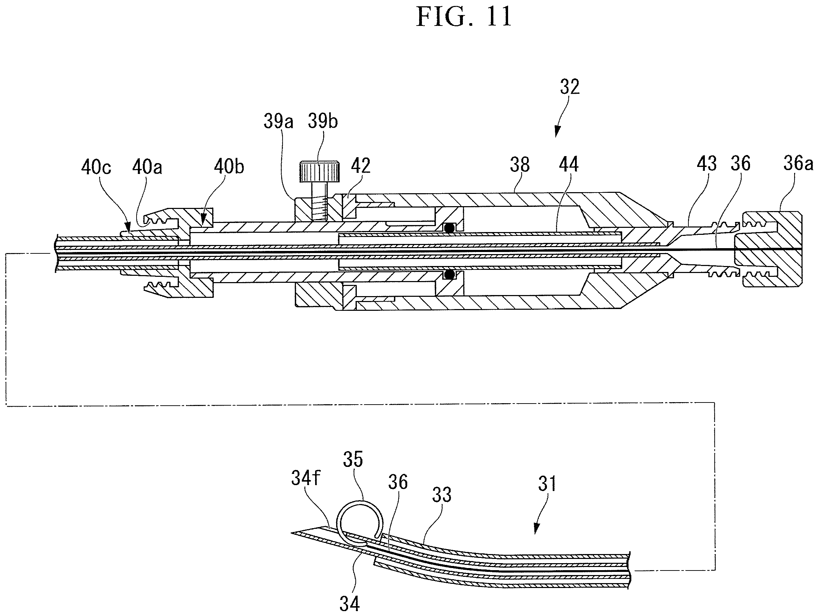

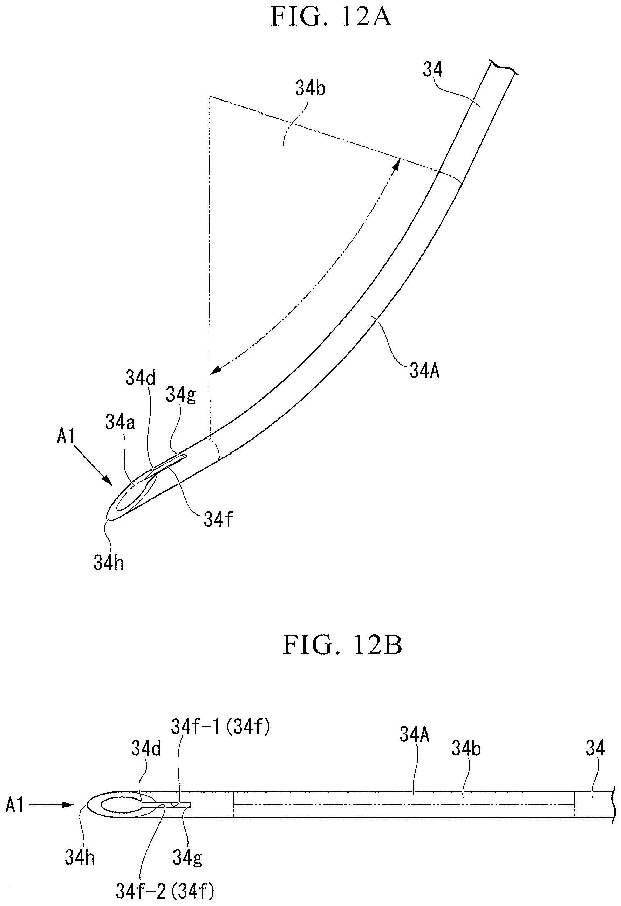

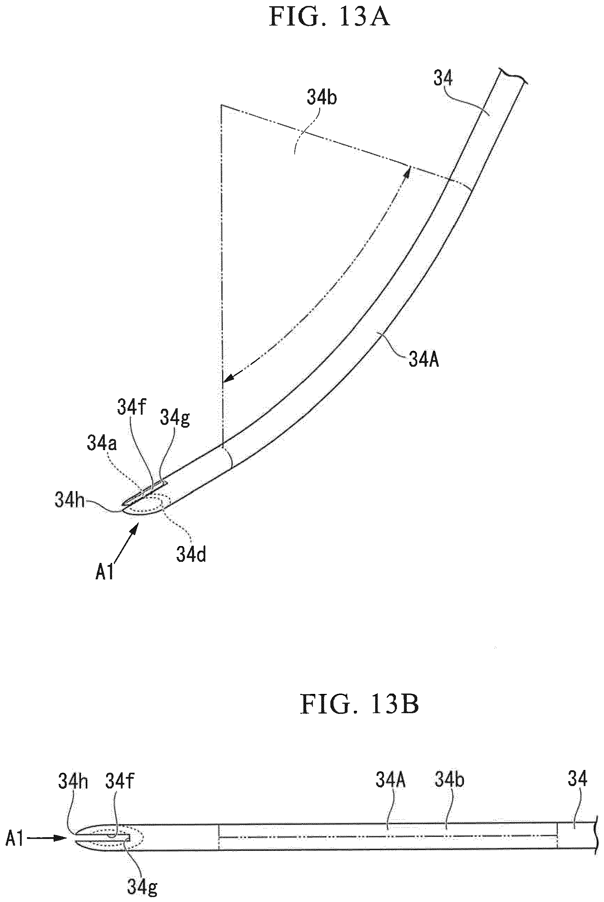

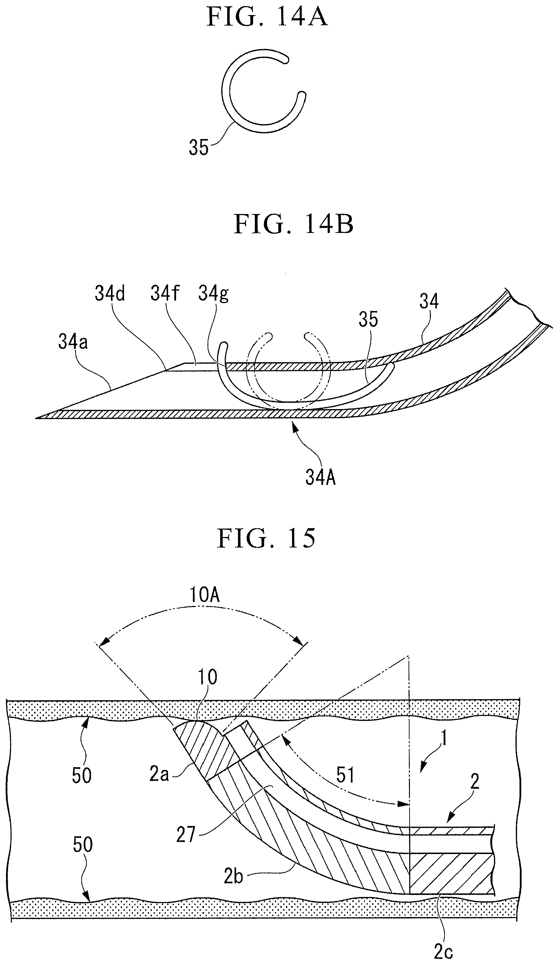

Next, the puncture needle for an ultrasound endoscope according to the present embodiment will be described using Figs. 9 to 14B. Fig. 9 is an overall external view of the puncture needle for an ultrasound endoscope. Figs. 10 and 11 are overall cross-sectional views. Figs. 12A, 12B, 13A, and 13B are explanatory views of a shape of a needle tube. Figs. 14A and 14B are explanatory views of an implant.

The puncture needle 30 for an ultrasound endoscope according to the present embodiment includes an insertion part 31 and an operation part 32. The insertion part 31 is a part to be inserted into the insertion channel 27 of the ultrasound endoscope 1. The operation part 32 is arranged at a proximal end part of the insertion part 31, and fixed to the treatment tool insertion port 3d of the ultrasound endoscope 1.

Parts of the insertion part 31 will be described.

A sheath 33 is a tube having flexibility, and positioned at the farthest distal end side of the insertion part 31. As a material for the sheath 33, for example, resin such as polyether ether ketone, polyether sulfone, and Teflon (registered trademark) is suitable. Alternatively, as a material for the sheath 33, a metal wire generally called a flexible shaft, and in particular, a metal obtained by winding a stainless steel wire into a coiled spring shape are suitable. The needle tube 34 is inserted into an inner cavity of the sheath 33. This structure can prevent the needle tube 34 from coming into direct contact with an inner surface of the insertion channel 27 to damage the needle tube 34 and the insertion channel 27.

The needle tube 34 is formed of a shape memory alloy which can be restored to a predetermined shape, a thin stainless steel pipe, or the like. A distal end part of the needle tube 34 is formed in a sharp shape. The needle tube 34 is inserted and arranged in the sheath 33 so as to advance and retract.

The vicinity of the distal end of the needle tube 34 in accordance with the present claimed invention, is shown in detail in Figs. 12A to 13B. In a natural state, at least a portion in the vicinity of the distal end of the needle tube 34 is worked into a smooth arc shape. Specifically, the needle tube 34 is provided with a curved shape part 34A that is formed in an arc shape in the absence of external force, deformed by the external force, and restored to the original arc shape when the external force is released. In Figs. 12A and 12B, the farthest distal end part of the needle tube 34 is not worked into the arc shape. However, the farthest distal end part may be included in a range to be worked into the arc shape.

The distal end of the needle tube 34 is formed in such a shape that the distal end is shaved off at an angle in the same way as a general syringe needle. An inner cavity is opened in a surface shaved off at an angle. This distal end opening 34a is formed in such a manner that a direction vertical to a distal end surface, that is, a direction in which the distal end opening 34a is viewed to be a maximum area (represented by an arrow A1 in Fig. 12A) is substantially in parallel with a plane 34b including a longitudinal central axis of the needle tube 34. A point 34d at the farthest proximal end side of the distal end opening 34a is on the same plane as the plane 34b.

A slit part (side hole) 34f is configured in such a manner that, among a tube wall of the needle tube 34 at a side more distal than the curved shape part 34A, a part of a tube wall is cut off, the tube wall crossing a plane including a central line of the curved shape part 34A, the tube wall being on the inside of a curve of the curved shape part 34A (side facing a curve center of the needle tube 34 in a curved state). The implant 35 can be delivered from the slit part 34f. Specifically, the slit part 34f extends, to the further proximal side, from the point 34d at the farthest proximal end side of the distal end opening 34a at the distal end of the needle tube 34. The slit part 34f is formed, in a longitudinal central axis direction of the needle tube 34, in an elongated hole shape larger than a diameter of the implant 35 which will be described later. In the present embodiment, a point 34g at the farthest proximal end side of the slit part 34f is on the same plane as the plane 34b. The slit part 34f may extend until the point 34g at the farthest proximal end side is positioned at the curved shape part 34A.

The opening width of the slit part 34f is set based on an external dimension of the implant 35. Specifically, the opening width of the slit part 34f has such a clearance that the implant 35 can advance and retract, and is larger than an external dimension of a wire constituting the implant 35.

In the example shown in Figs. 13A and 13B, the distal end of the needle tube 34 is shaved off in the reverse direction of the example shown in Figs. 12A and 12B. In this case, the slit part 34f is formed in such a shape that the tube wall of the needle tube 34 is cut off, from a point 34h at the farthest distal end side of the distal end opening 34a, in an elongated hole shape in the longitudinal central axis direction of the needle tube 34. In this case as well, the point 34g at the farthest proximal end side of the slit part 34f is on the same plane as the plane 34b, and positioned at the curved shape part 34A.

Any of the configuration shown in Figs. 12A and 12B and the configuration shown in Figs. 13A and 13B may be selected depending on the purpose.

The implant 35 is a piece of metal containing a substance that generates very weak radiation for treatment. The implant 35 is shown in detail in Figs. 14A and 14B. The implant 35 has such a shape that a wire thinner than the inner cavity of the needle tube 34 is bent. The implant 35 is a rod spring having elasticity or an elastic wire having a coil shape. In a straight state, therefore, the implant 35 has restoring force to restore the implant 35 to a wind shape. Since the implant 35 loaded into the inner cavity of the needle tube 34 at a position near a distal end of the inner cavity has elasticity, the implant 35 always pushes an inner wall of the needle tube 34 with the force to return to the original shape. Therefore, the implant 35 is not easily removed from the needle tube 34 to the outside.

A stylet 36 is a thin elongated wire. A material for the stylet 36 is, for example, stainless steel or nickel titanium. The stylet 36 is arranged at a proximal end side of the inner cavity of the needle tube 34 so as to be inserted into and removed from the needle tube 34. The stylet 36 is a releasing mechanism that pushes the implant 35 out of the needle tube 34.

Components of the operation part 32 will be described.

An operation part main body 37 is formed of a resin material.

A slider 38 is provided so as to slide against the operation part main body 37. The slider 38 is formed of a resin material.

A stopper 39 is a member capable of setting a sliding distance of the slider 38 against the operation part main body 37 to a desired value depending on a measurement result, and configured as follows. A stopper member 39a is arranged so as to slide against the operation part main body 37. A material for the stopper member 39a is formed of, for example, resin. A fixing screw (stopper screw) 39b is arranged to be screwed with the stopper member 39a, and fixes the stopper member 39a to a desired position. A material for the fixing screw 39b is made of metal or a hard resin.

The operation part main body 37 is formed in a thin elongated pipe shape, a proximal end part of which is provided with a flange part 37a. A connection part 40 made of resin is stuck and fixed to a distal end part of the operation part main body 37. A proximal end part of the sheath 33 is fixedly provided on the connection part 40. A screw 40a connected and fixed to the treatment tool insertion port 3d of the ultrasound endoscope 1 is formed at a distal end side of the connection part 40. A recessed part 40b in which the distal end part of the operation part main body 37 is arranged is formed at a proximal end part of the connection part 40. The sheath 33 is fixed to a distal end connection part 40c formed at the connection part 40.

A recessed part is formed in an inner peripheral surface of the flange part 37a. An O ring 41 holding a guide pipe which will be described later is arranged in the recessed part of the flange part 37a. A notch stepped portion 37b that has a plane part on which a distal end surface of the fixing screw 39b abuts is formed at a predetermined position on an outer peripheral surface at an end side more distal than the flange part 37a.

When the puncture needle 30 for an ultrasound endoscope is manufactured and shipped, the distal end surface of the fixing screw 39b abuts on the plane part of the notch stepped portion 37b with predetermined torque. This allows the slider 38 to be arranged at the proximal end side of the operation part main body 37.

In this arrangement state, distal end parts of the needle tube 34 and the stylet 36 are arranged within the sheath 33. If the slider 38 is affected by some external force and moved to a distal end side, a side part of the fixing screw 39b abuts on a raised part of the notch stepped portion 37b to stop the movement of the slider 38 to the distal end side. Needless to say, the distal end parts of the needle tube 34 and the stylet 36 do not project from a distal end of the sheath 33 in this abutting state.

When the stopper screw 39b is loosened, the stopper member 39a is allowed to slide and move on the operation part main body 37 in a longitudinal direction. The stopper member 39a is caused to slide and move to an arbitrary position, and the fixing screw 39b is screwed into the stopper member 39a to fix the stopper member 39a, whereby a maximum movable distance of the slider is set.

The slider 38 is formed in a pipe shape, a proximal end part of which is provided with a small diameter part 38a. A sliding arrangement member 42 for arranging the slider 38 such that the slider 38 can slide against the operation part main body 37 is stuck and fixed to the distal end part of the slider 38.

In addition, a ferrule member 43 made of resin is arranged at an opening part at the proximal end part of the slider 38. A proximal end part of the needle tube 34 and a proximal end part of the guide pipe 44, a distal end part of which is held by the O ring 41, are fixed to a distal end part of the ferrule member 43. A proximal end part of the ferrule member 43 is formed in a luer-lock shape capable of connecting a syringe or the like.

The stylet 36 is inserted from the ferrule member 43 of the slider 38. A lug 36a made of resin is integrally provided at a proximal end part of the stylet 36.

After the components are assembled, the puncture needle 30 for an ultrasound endoscope configured in the above-mentioned manner is housed in a sterilization bag (not shown) and disinfected.

Operation of the disposable puncture needle 30 for an ultrasound endoscope configured in the above-mentioned manner will be described. First, a combination of the ultrasound endoscope 1 shown in Figs. 1 to 4 and the needle tube 34 shown in Figs. 12A and 12B will be described. Next, it will be described that the ultrasound endoscope 100 shown in Figs. 5 to 8 and the needle tube 34 shown in Figs. 13A and 13B can be used to achieve exactly the same function.

First, the puncture needle 30 for an ultrasound endoscope housed in a sterilization bag (not shown) is taken out of the sterilization bag. The implant 35 is loaded in advance into the needle tube 34 of the puncture needle 30 for an ultrasound endoscope. In the straight state, the implant 35 is inserted from the distal end opening 34a of the needle tube 34 into the needle tube 34. Alternatively, the implant 35 may be inserted from the proximal end of the needle tube 34 and delivered to the distal end by the stylet 36.

While loading the implant 35 into the needle tube 34, it is not easy to direct an orientation of the implant 35 in a circumferential direction of the needle tube 34 to a specific orientation and simultaneously insert the implant 35 into the needle tube 34. In addition, the wire constituting the implant 35 might be somewhat twisted during the process of inserting the implant 35 into the needle tube 34. In the present embodiment, regardless of the orientation of the implant 35 in the circumferential direction while being inserted into the needle tube 34, when the implant 35 is arranged such that a distal end of the implant 35 is positioned at the curved shape part 34A of the needle tube 34, the implant 35 rotates within the needle tube 34 such that a wind direction of the implant 35 follows a curved direction of the curved shape part 34A. Therefore, after the rotation of the implant 35, the distal end of the implant 35 is positioned within the plane 34b including the longitudinal central axis of the needle tube 34. Specifically, the implant 35 is arranged with respect to the needle tube 34 such that the distal end of the implant 35 can reach the point 34g at the farthest proximal end side of the slit part 34f when the implant 35 is pushed by the stylet 36 within the needle tube 34.

When a part of the implant 35 is positioned at the curved shape part 34A, the implant 35 receives, from the inner surface of the needle tube 34, force to rotate to follow the curved direction of the curved shape part 34A. In addition, when the distal end of the implant 35 is positioned at the curved shape part 34A, the position of the distal end of the implant 35 that enters the slit part 34f first is easily moved to a suitable position.

Next, the sheath 33 is inserted from the treatment tool insertion port 3d of the ultrasound endoscope 1 into the insertion channel 27. The screw 40a provided at the connection part 40 of the operation part 32 is screwed with the treatment tool insertion port 3d to fix the puncture needle 30 for an ultrasound endoscope to the ultrasound endoscope 1.

An ultrasound image of the distal end part of the sheath 33 is clearly drawn on an ultrasound observing image on which a target site is displayed. In this case, a positional relation between the distal end of the sheath 33 and the target site is set. After that, the distance between the distal end of the sheath 33 and the target site is measured.

Next, the fixing screw 39b is loosened, and the stopper member 39a is caused to slide and move on the operation part main body 37 so as to correspond to the above-mentioned distance. When the stopper member 39a is moved to a predetermined position, the fixing screw 39b is fastened.

After that, an operator grips the slider 38 and quickly moves the slider 38 toward the stopper 39. As a result, the distal end of the needle tube 34 infallibly punctures the target site.

When the needle tube 34 is confirmed to have reached the target site, the lug 36a of the stylet 36 is pushed into a distal end side. This allows the stylet 36 to move to the distal end side, and the implant 35 is discharged from the distal end opening 34a at the distal end of the needle tube 34 to be placed within a body.

In order to place the implant 35 accurately at the target site, the implant 35 needs to be discharged while being monitored on an ultrasound observing image. In the embodiment of the present invention, therefore, an angular position around an axis of the needle tube 34 is controlled to cause a direction in which the implant 35 is discharged to correspond to the ultrasound observation plane. Hereinafter, a method for causing the direction in which the implant 35 is discharged to correspond to the ultrasound observation plane will be described.

Since ultrasound is significantly attenuated in the air, the ultrasound transducer part 10 arranged at the distal end of the ultrasound endoscope 1 needs to be brought into close contact with a tissue within a body during the observing of an ultrasound image. In Fig. 15, since the ultrasound endoscope 1 is inserted into a body lumen tissue 50, the bendable part 2b of the insertion part 2 of the endoscope needs to be bent in a direction generally called an up direction to be directed to the body lumen tissue 50 in order to bring the ultrasound transducer part 10 into contact with the tissue. Since the bendable part 2b is bent and results in a substantially arc shape, the insertion channel 27 arranged within the bendable part 2b is also inevitably formed in a substantially arc shape. At this time, a plane 51 including a longitudinal central axis of the insertion channel 27 is substantially the same plane as the ultrasound observation plane 10A.

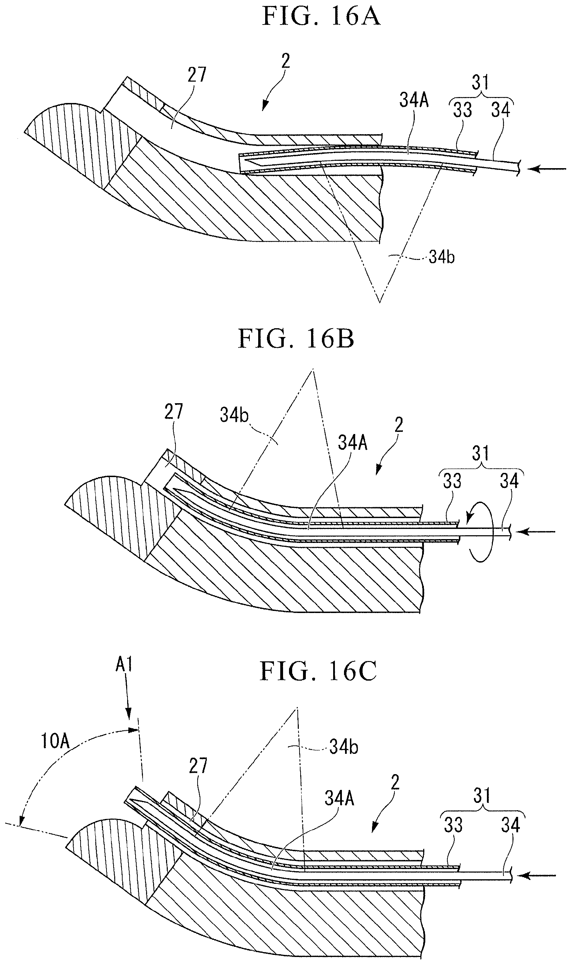

In Figs. 16A to 16C, the insertion part 31 of the puncture needle 30 for an ultrasound endoscope, which includes the needle tube 34, a portion in the vicinity of the distal end of which is curved in the smooth arc shape as mentioned above, passing through the bent insertion channel 27 is shown in time series order of operation. In Fig. 16A, the distal end of the insertion part 31 is pushed to advance to the edge of the bendable part of the insertion channel 27 of the ultrasound endoscope 1. When the insertion part 31 is pushed to advance to the further distal end side, the arc shape of the needle tube 34 reaches the bent shape of the insertion part 2 as shown in Fig. 16B. Since the needle tube 34 receives the force from the inner wall of the insertion channel 27 due to the insertion, the needle tube 34 is rotated around a longitudinal axis such that the arc shape of the insertion channel 27 and the arc shape of the needle tube 34 are positioned on the same plane (including substantially the same plane). As a result, the plane 34b including the longitudinal central axis of the needle tube 34 (i.e. plane including an axial line that coincides with an orientation of the distal end opening of the needle tube 34) becomes substantially the same as the plane 51 including the longitudinal central axis of the insertion channel 27. Therefore, the plane 34b becomes substantially the same plane as the ultrasound observation plane 10A.

Even in a case where the wind direction of the implant 35 does not follow the curved direction of the curved shape part 34A of the needle tube 34 before the needle tube 34 is inserted into the insertion channel 27, once the curved shape part 34A is arranged within the bent insertion channel 27, the implant 35 rotates within the needle tube 34 such that the wind shape of the implant 35 follows the curved shape of the needle tube 34 by means of the restoring force to restore the implant 35 to the coil shape. Specifically, the curved shape part 34A of the needle tube 34 rotationally moves until the curved shape part 34A of the needle tube 34 follows the arc shape of the insertion channel 27, and the implant 35 rotationally moves until the wind shape of the implant 35 follows the curved shape part 34A of the needle tube 34.

In Fig. 16C, the insertion part 31 that has reached a predetermined position is shown. Although the angular position around the axis of the needle tube 34 does not change from that of Fig. 16B, the angular position around the axis of the needle tube 34 becomes more stable since an overlapping length of the arc shape of the needle tube 34 and the bent shape of the insertion part 2 is increased. Since the direction A1 in which the distal end opening 34a is viewed to be the maximum area is substantially in parallel with the plane 34b, the direction A1 is substantially in parallel with the ultrasound observation plane 10A. In other words, the axial line that coincides with the orientation of the distal end opening of the needle tube 34 in the direction A1 is substantially in parallel with the ultrasound observation plane 10A.

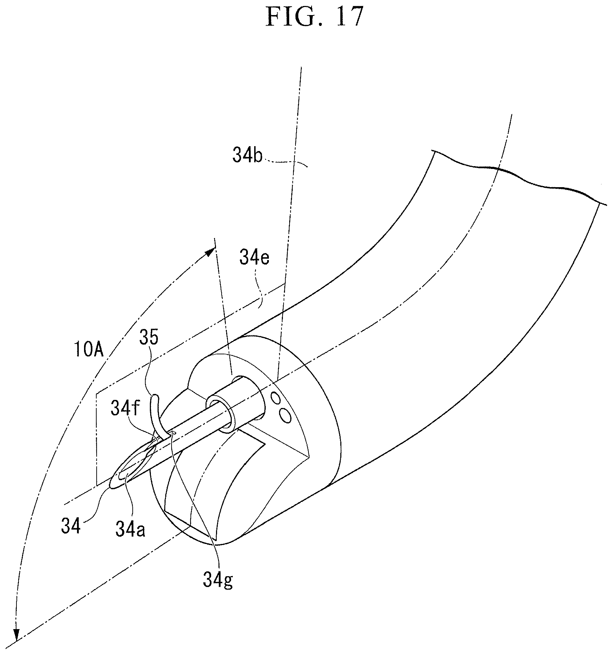

It has already been stated that the implant 35 has elasticity and is in the straight state when housed in the needle tube 34. As shown in Fig. 17, when the implant 35 is discharged from the distal end opening 34a of the needle tube 34, the distal end of the implant 35 is delivered from the point 34g at the farthest proximal side of the slit part 34f to the outside of the needle tube 34. Therefore, an outer surface of the implant 35 is delivered along the plane 34b including the longitudinal central axis of the needle tube 34 while being supported by the slit part 34f. Specifically, in the present embodiment, the implant 35 is always within the plane 34b including the longitudinal central axis of the needle tube 34 without operation to project in a direction crossing the plane 34b including the longitudinal central axis of the needle tube 34.

The implant 35 is supported so as to be sandwiched between a pair of wall surfaces 34f-1, 34f-2 (refer to Fig. 12B) facing each other at the slit part 34f. The implant 35 is further supported by an inner peripheral surface of the needle tube 34 which is a surface located at a position facing the slit part 34f when the needle tube 34 is viewed in a longitudinal central axis direction of the needle tube 34. Therefore, when the implant 35 is delivered through the slit part 34f of the needle tube 34, the implant 35 is delivered along a plane 34e and hardly moves in a direction crossing the plane 34e.

The implant 35 is discharged on the plane 34e. In this case, the plane 34e is substantially the same as the plane 34b and includes the proximal end point 34d. Since the plane 34b is substantially the same as the ultrasound observation plane 10A, the implant 35 can be suitably monitored on an ultrasound image.

Next, use of the ultrasound endoscope 100 shown in Figs. 5 to 8 and the needle tube 34 shown in Figs. 13A and 13B will be described.

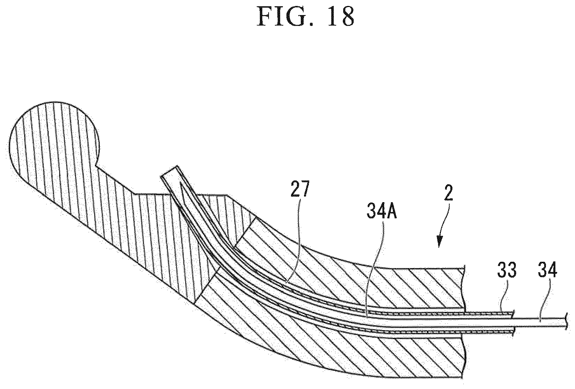

Fig. 18 shows a state where the insertion part 31 of the puncture needle 30 for an ultrasound endoscope, which includes the needle tube 34, a portion in the vicinity of the distal end of which is curved in the smooth arc shape in a natural state, has passed through the bent insertion channel 27 and reached a predetermined position.

As shown in Fig. 6, the insertion channel outlet 124 is provided so as to be inclined by the angle α with respect to the longitudinal axis direction of the hard part 102a such that the guided treatment tool does not come into contact with the large ultrasound transducer part 110. As shown in Fig. 15, when a surgical technique is practiced, the bendable part 2b of the endoscope insertion part 2 is bent in the direction generally called the up direction. At this time, it can be understood that a lumen formed by the insertion channel outlet 124 and the insertion channel 27 can let the needle tube 34 curved in the arc shape smoothly pass therethrough. The implant 35 enters the slit part 34f from a proximal end of the slit part 34f extending to a proximal side from the point 34h at the farthest distal end side of the distal end opening 34a of the needle tube 34. The implant 35 is then ejected from the needle tube 34 to the outside while keeping a positional relation that allows the implant 35 to be monitored on an ultrasound image.

Therefore, when the implant 35 is discharged from the distal end opening 34a of the needle tube 34, the implant 35 can be suitably monitored on an ultrasound image.

As a matter of course, use of the ultrasound endoscope 1 shown in Figs. 1 to 4 in combination with the needle tube shown in Figs. 13A and 13B and use of the ultrasound endoscope 100 shown in Figs. 5 to 8 in combination with the needle tube shown in Figs. 12A and 12B also achieve the same function. When the release of the implant 35 into a body is finished, the puncture needle 30 for an ultrasound endoscope is removed from the ultrasound endoscope and discarded. A series of surgical techniques is thus finished.

As mentioned above, according to the puncture needle 30 for an ultrasound endoscope according to the present embodiment, a position of the implant 35 released from the needle tube 34 can always be recognized by the ultrasound endoscope 1 from the first time the implant 35 is delivered from the needle tube 34. By using the puncture needle 30 for an ultrasound endoscope according to the present embodiment, therefore, it is possible to proceed with the surgical technique while recognizing a position of the distal end of the implant 35. Thus, the possibility that the distal end of the implant 35 comes into contact with a living tissue outside a field of view of the ultrasound endoscope 1 can be suppressed to a low level.

### (Second Embodiment) - not forming part of the invention

A second embodiment is different from the first embodiment in a configuration of a distal end shape of a needle tube.

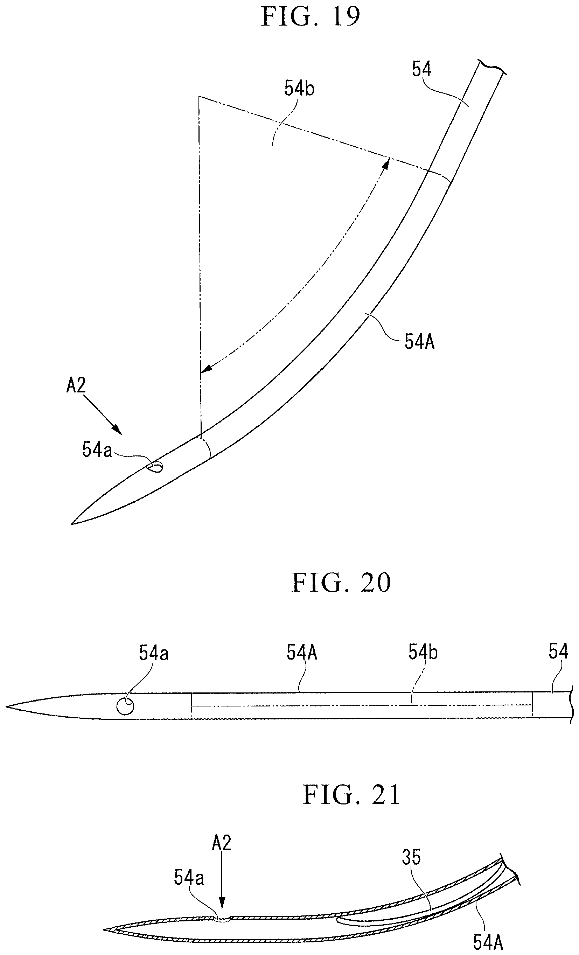

As shown in Figs. 19 to 21, a distal end of a needle tube 54 is sharp, a side surface of which is provided with an opening 54a. This opening 54a is formed in such a manner that a direction in which the opening 54a is viewed from the front, that is, a direction in which the opening 54a is viewed to be a maximum area (represented by an arrow A2 in Figs. 19 and 21) is substantially in parallel with a plane 54b including a longitudinal central axis of the needle tube 54. Only a single opening 54a is provided in the present embodiment. The opening 54a is a path for ejecting the implant 35 from the inside of the needle tube 54 to the outside, and corresponds to the slit part 34f described in the above-mentioned first embodiment. In the present embodiment, a curved shape part 54A formed in a curved shape same as the first embodiment is provided at a distal part of the needle tube 54.

Even in this configuration, the implant 35 is pushed out of the opening 54a by the stylet 36, whereby the implant 35 can be observed through the ultrasound endoscope 1.

The embodiment of the present invention and the second embodiment have been described in detail so far with reference to the drawings. However, the specific configuration is not limited to the embodiments, and design changes or the like within a range not departing from the scope of the present invention are also included.

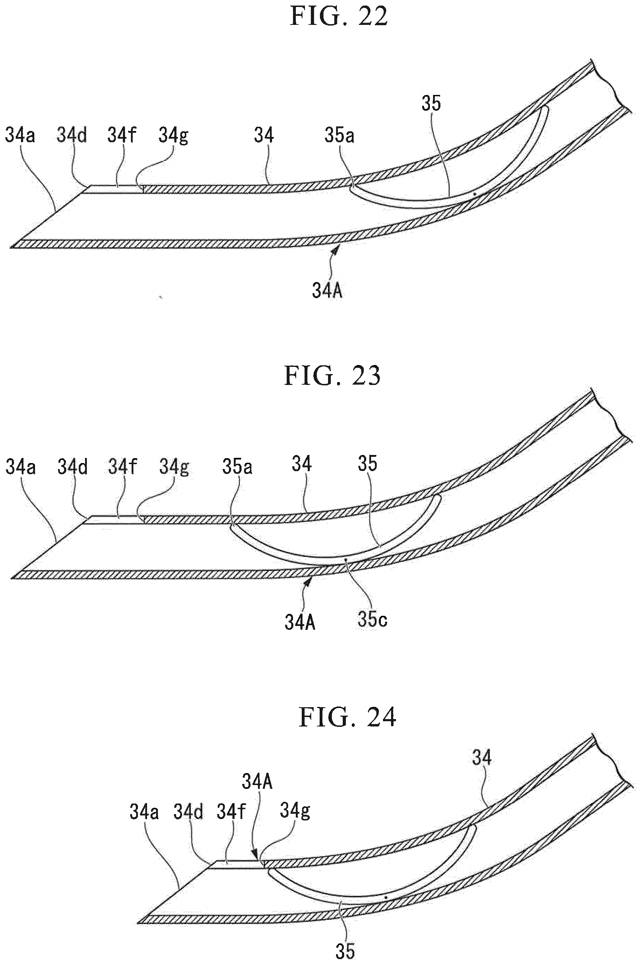

Figs. 22 to 24 are partial cross-sectional views showing exemplary design changes for the above-mentioned embodiments.

For example, as shown in Fig. 22, when the implant 35 made of a wire is stretched and loaded into the needle tube 34, a distal end 35a of the wire constituting the implant 35 may be positioned at the curved shape part 34A.

As shown in Fig. 23, when the implant 35 made of a wire is stretched and loaded into the needle tube 34, a central part 35c of the implant 35 in a straight state may be positioned at the curved shape part 34A.

As shown in Fig. 24, the point 34g at the proximal end of the slit part 34f may be positioned at the curved shape part 34A.

While the preferred embodiment of the present invention and the second embodiment have been described above, the present invention is not limited to these embodiments and their modifications. Configurations can be added, omitted, replaced, and otherwise changed within a range not departing from the scope of the present invention.

The present invention is not limited by the aforementioned description, and is only limited by the scope of appended claims.

### Industrial Applicability

According to the above-mentioned aspects, a position of a tool released from a puncture needle can be recognized by an ultrasound endoscope.

### Reference Signs List

1, 100: ultrasound endoscope
2: insertion part (endoscope insertion part)
2a, 102a: hard part
2b: bendable part
2c: flexible tube part
3: operation part
3a: angle knob
3d: treatment tool insertion port
4: universal cord
5: endoscope connector
6: ultrasound cable
7: ultrasound connector
10, 110: ultrasound transducer part
10A, 110A: ultrasound observation plane
21: distal end surface
22: observing window
23: illumination window
24, 124: insertion channel outlet
27: treatment tool insertion channel
30: puncture needle for ultrasound endoscope
31: insertion part
32: operation part
33: sheath
34: needle tube
34a: distal end opening
34A, 54A: curved shape part
34b: plane
34d: point
34e: plane
34f: slit part
34g: point
34h: point
35: implant
36: stylet
36a: lug
37: operation part main body
37a: flange part
37b: notch stepped portion
38: slider
38a: small diameter part
39: stopper
39a: stopper member
39b: fixing screw (stopper screw)
40: connection part
40a: screw
40b: recessed part
40c: distal end connection part
41: ring
42: sliding arrangement member
43: ferrule member
44: guide pipe
50: body lumen tissue
51: plane
54: needle tube
54a: opening
54b: plane

## Claims

1. A puncture needle (30) configured to be inserted in an ultrasound endoscope (1, 100) having a bendable part (2b) capable of being operated to be bent, the ultrasound endoscope (1, 100) being configured such that a central axis extending from an outlet (24, 124) of an insertion channel (27) substantially coincides with an ultrasound observation plane (10A, 110A), the puncture needle (30) comprising:
a sheath (33) configured to be freely advanced or retracted in the insertion channel (27), the sheath (33) being configured to be capable of being inserted into the insertion channel (27);
a needle tube (34) having an outer surface and an inner surface that forms an internal space extending along a longitudinal axis of the needle tube (34), the needle tube (34) having a distal end opening (34a) which is in communication with the internal space at a distal end of the needle tube (34), the needle tube (34) being capable of being inserted into the sheath (33);
a wire (35) having a proximal end and a distal end, the wire (35) having elasticity such that the wire (35) is restored to a wind shape in an absence of external force, the wire (35) being stretched and loaded into the internal space of the needle tube (34) against restoration to the wind shape such that the wire (35) is supported by portions of the inner surface of the needle tube (34) which are spaced apart from one another in a direction of the longitudinal axis of the needle tube (34);
a slit part (34f) formed to open from the inner surface to the outer surface of the needle tube (34), the slit part (34f) having a pair of wall surfaces (34f-1, 34f-2) which are spaced apart from one another, the slit part (34f) having an opening width larger than an external diameter of the wire (35); and
a stylet (36) configured to push the proximal end of the wire (35) loaded within the needle tube (34), wherein
the needle tube (34) has a needle tube curved shape part (34A) formed at a distal side of the needle tube (34), the needle tube curved shape part (34A) having elasticity such that the needle tube curved shape part (34A) is restored to a curved shape in an absence of external force,
the needle tube (34) has a needle tube distal part having a needle tip (34h) formed to be sharpened to puncture a tissue within a body cavity, the needle tube distal part being formed at a side more distal than the needle tube curved shape part (34A),
a section of the inner surface facing the slit part (34f) supports the wire (35) within the needle tube (34), such that the wire (35) is restored to the wind shape in the ultrasound observation plane (10A, 110A),
the slit part (34f) is formed at the needle tube distal part such that the slit part (34f) is in communication with the distal end opening (34a) of the needle tube (34), and opens toward an inside of the curved shape of the needle tube curved shape part (34A), and
in a state that the needle tube (34) projects from the insertion channel (27) and the distal end of the wire (35) is pushed from the slit part (34f) by the stylet (36), the pair of wall surfaces (34f-1, 34f-2) support the wire (35) such that the wire (35) is sandwiched between the pair of wall surfaces (34f-1, 34f-2).

2. The puncture needle (30) according to claim 1, wherein
each of the portions of the inner surface of the needle tube (34) supporting the wire (35) is positioned on a prescribed plane.

3. The puncture needle (30) according to claim 1, wherein
in a state that the distal end of the wire (35) is arranged inside the needle tube curved shape part (34A) positioned in the insertion channel (27), as the insertion channel (27) is bent by the bendable part (2b), the needle tube curved shape part (34A) follows a curve of the insertion channel (27), and the wire (35) receives a force from the inner surface of the needle tube (34) in the needle tube curved shape part (34A), so that the wire (35) rotates such that the wind shape of the wire (35) follows the curved shape of the needle tube curved shape part (34A), and the distal end of the wire (35) is positioned on the ultrasound observation plane (10A, 110A).

4. The puncture needle (30) according to claim 1, wherein
in a state that the wire (35) is supported by the portions of the inner surface of the needle tube (34) which are spaced apart from one another in the direction of the longitudinal axis of the needle tube (34), the portions supporting the wire (35) are positioned on a plane which includes the slit part (34f) and which is along a central axis of the needle tube (34), and the portions hold the wire (35) between a proximal end of the slit part (34f) and the stylet (36).

5. The puncture needle (30) according to claim 2, wherein
in a state that the distal end of the wire (35) is restored to the wind shape in the ultrasound observation plane (10A, 110A) and the pair of wall surfaces (34f-1, 34f-2) support the wire (35) such that the wire (35) is sandwiched between the pair of wall surfaces (34f-1, 34f-2), the proximal end of the wire (35) and a central part between the distal end and the proximal end of the wire (35) are positioned on the prescribed plane.

6. The puncture needle (30) according to claim 2, wherein
the distal end, the proximal end, and a central part between the distal end and the proximal end of the wire (35) are positioned on the prescribed plane.

7. The puncture needle (30) according to claim 1, wherein
the distal end of the wire (35) is positioned at the needle tube curved shape part (34A) in a state that the wire (35) is stretched and loaded into the needle tube (34).

8. The puncture needle (30) according to claim 1, wherein
a central part of the wire (35) is positioned at the needle tube curved shape part (34A) in a state that the wire (35) is stretched and loaded into the needle tube (34).

9. The puncture needle (30) according to claim 1, wherein
a proximal end of the slit part (34f) is positioned at the needle tube curved shape part (34A).

## Patentansprüche

1. Punktionsnadel (30), die konfiguriert ist, um in ein Ultraschallendoskop (1, 100) eingeführt zu werden, mit einem biegsamen Teil (2b), der zum Biegen betrieben werden kann, wobei das Ultraschallendoskop (1, 100) so konfiguriert ist, dass eine Mittelachse, die sich von einem Ausgang (24, 124) eines Einführkanals (27) erstreckt, im Wesentlichen mit einer Ultraschallbeobachtungsebene (10A, 110A) übereinstimmt, wobei die Punktionsnadel (30) umfasst:
eine Hülle (33), die konfiguriert ist, um in dem Einführkanal (27) frei vor- oder zurückgeschoben zu werden, wobei die Hülle (33) konfiguriert ist, um in den Einführkanal (27) eingeführt werden zu können;
ein Nadelrohr (34) mit einer Außenfläche und einer Innenfläche, die einen sich entlang einer Längsachse des Nadelrohrs (34) erstreckenden Innenraum bildet, wobei das Nadelrohr (34) eine distale Endöffnung (34a) aufweist, die mit dem Innenraum an einem distalen Ende des Nadelrohrs (34) in Verbindung steht, wobei das Nadelrohr (34) in die Hülle (33) einsetzbar ist;
ein Kabel (35) mit einem proximalen Ende und einem distalen Ende, wobei das Kabel (35) eine solche Elastizität aufweist, dass das Kabel (35) in Abwesenheit von äußerer Kraft wieder in eine gewundene Form gebracht wird, wobei das Kabel (35) gestreckt und in den Innenraum des Nadelrohrs (34) gegen Wiederherstellung der gewundenen Form geladen wird, so dass das Kabel (35) durch Abschnitte der Innenfläche des Nadelrohrs (34) getragen wird, die in Richtung der Längsachse des Nadelrohrs (34) voneinander beabstandet sind;
ein Schlitzteil (34f), das so ausgebildet ist, dass es sich von der Innenfläche zur Außenfläche des Nadelrohrs (34) öffnet, wobei das Schlitzteil (34f) ein Paar von Wandflächen (34f-1, 34f-2) aufweist, die voneinander beabstandet sind, wobei das Schlitzteil (34f) eine Öffnungsweite aufweist, die größer ist als ein Außendurchmesser des Kabels (35); und
ein Mandrin (36), das konfiguriert ist, um das proximale Ende des Kabels (35), das in das Nadelrohr (34) eingelegt ist, zu drücken, wobei
das Nadelrohr (34) einen gekrümmten Formteil (34A) aufweist, der an einer distalen Seite des Nadelrohrs (34) ausgebildet ist, wobei der gekrümmte Formteil (34A) des Nadelrohrs eine solche Elastizität aufweist, dass der gekrümmte Formteil (34A) des Nadelrohrs in Abwesenheit von externer Kraft wieder in eine gekrümmte Form gebracht wird,
das Nadelrohr (34) einen distalen Teil des Nadelrohrs mit einer Nadelspitze (34h) aufweist, die zum Schärfen ausgebildet ist, um ein Gewebe innerhalb einer Körperhöhle zu durchbohren, wobei der distale Teil des Nadelrohrs an einer Seite ausgebildet ist, die weiter distal ist als der gekrümmte Formteil (34A) des Nadelrohrs,
ein Abschnitt der Innenfläche, der dem Schlitzteil (34f) zugewandt ist, das Kabel (35) innerhalb des Nadelrohrs (34) trägt, so dass das Kabel (35) in der Ultraschallbeobachtungsebene (10A, 110A) wieder in die gewundene Form gebracht wird,
das Schlitzteil (34f) am distalen Teil des Nadelrohrs so ausgebildet ist, dass das Schlitzteil (34f) in Verbindung mit der distalen Endöffnung (34a) des Nadelrohrs (34) steht und sich zu einer Innenseite der gekrümmten Form des gekrümmten Formteils (34A) öffnet, und
in einem Zustand, in dem das Nadelrohr (34) aus dem Einführkanal (27) ragt und das distale Ende des Kabels (35) durch das Mandrin (36) aus dem Schlitzteil (34f) geschoben wird, das Paar von Wandflächen (34f-1, 34f-2) das Kabel (35) so trägt, dass das Kabel (35) zwischen dem Paar von Wandflächen (34f-1, 34f-2) sandwichartig angeordnet ist.

2. Punktionsnadel (30) nach Anspruch 1, wobei
jeder der Abschnitte der Innenfläche des Nadelrohrs (34), das das Kabel (35) trägt, auf einer vorgegebenen Ebene positioniert ist.

3. Punktionsnadel (30) nach Anspruch 1, wobei
in einem Zustand, in dem das distale Ende des Kabels (35) innerhalb des im Einführkanal (27) positionierten gekrümmten Formteils (34A) des Nadelrohrs angeordnet ist, da der Einführkanal (27) durch das biegsame Teil (2b) gebogen wird, wobei das gekrümmte Formteil (34A) des Nadelrohrs einer Kurve des Einführkanals (27) folgt, und das Kabel (35) eine Kraft von der Innenfläche des Nadelrohrs (34) in dem gekrümmten Formteil (34A) des Nadelrohrs aufnimmt, so dass sich das Kabel (35) so dreht, dass die gewundene Form des Kabels (35) der gekrümmten Form des gekrümmten Formteils (34A) des Nadelrohrs folgt, und das distale Ende des Kabels (35) auf der Ultraschallbeobachtungsebene (10A, 110A) positioniert ist.

4. Punktionsnadel (30) nach Anspruch 1, wobei
in einem Zustand, in dem das Kabel (35) von den Abschnitten der Innenfläche des Nadelrohrs (34) getragen wird, die in Richtung der Längsachse des Nadelrohrs (34) voneinander beabstandet sind, die Abschnitte, die das Kabel (35) tragen, auf einer Ebene positioniert sind, die das Schlitzteil (34f) beinhaltet und die sich entlang einer Mittelachse des Nadelrohrs (34) befindet, und die Abschnitte das Kabel (35) zwischen einem proximalen Ende des Schlitzteils (34f) und dem Mandrin (36) halten.

5. Punktionsnadel (30) nach Anspruch 2, wobei
in einem Zustand, in dem das distale Ende des Kabels (35) in der Ultraschallbeobachtungsebene (10A, 110A) wieder in die gewundene Form gebracht wird und das Paar von Wandflächen (34f-1, 34f-2) das Kabel (35) so trägt, dass das Kabel (35) zwischen dem Paar von Wandflächen (34f-1, 34f-2), dem proximalen Ende des Kabels (35) und einem zentralen Teil zwischen dem distalen Ende und dem proximalen Ende des Kabels (35) auf der vorgeschriebenen Ebene angeordnet ist.

6. Punktionsnadel (30) nach Anspruch 2, wobei
das distale Ende, das proximale Ende und ein zentraler Teil zwischen dem distalen Ende und dem proximalen Ende des Kabels (35) auf der vorgeschriebenen Ebene positioniert sind.

7. Punktionsnadel (30) nach Anspruch 1, wobei
das distale Ende des Kabels (35) an dem gekrümmten Formteil (34A) des Nadelrohrs in einem Zustand positioniert ist, in dem das Kabel (35) gestreckt und in dem Nadelrohr (34) geladen ist.

8. Punktionsnadel (30) nach Anspruch 1, wobei
ein zentraler Teil des Kabels (35) an dem gekrümmten Formteil (34A) des Nadelrohrs in einem Zustand positioniert ist, in dem das Kabel (35) gestreckt und in dem Nadelrohr (34) geladen ist.

9. Punktionsnadel (30) nach Anspruch 1, wobei
ein proximales Ende des Schlitzteils (34f) an dem gekrümmten Formteil (34A) des Nadelrohrs positioniert ist.

## Revendications

1. Aiguille de ponction (30) configurée pour être inséré dans un endoscope à ultrasons (1, 100) comportant une partie flexible (2b) pouvant être actionnée pour être courbée, l'endoscope à ultrasons (1, 100) étant configuré d'une manière telle qu'un axe central s'étendant à partir d'une sortie (24, 124) d'un canal d'insertion (27) coïncide sensiblement avec un plan d'observation ultrasonore (10A, 110A), l'aiguille de ponction (30) comprenant :
une gaine (33) configurée pour être avancée ou rétractée librement dans le canal d'insertion (27), la gaine (33) étant configurée pour pouvoir être insérée dans le canal d'insertion (27) ;
un tube d'aiguille (34) comportant une surface extérieure et une surface intérieure qui forme un espace interne s'étendant le long d'un axe longitudinal du tube d'aiguille (34), le tube d'aiguille (34) comportant une ouverture d'extrémité distale (34a) qui est en communication avec l'espace interne au niveau d'une extrémité distale du tube d'aiguille (34), le tube d'aiguille (34) pouvant être inséré dans la gaine (33) ;
un câble (35) comportant une extrémité proximale et une extrémité distale, le câble (35) présentant une élasticité telle que le câble (35) est rétabli à une forme enroulée en l'absence d'une force externe, le câble (35) étant étiré et chargé dans l'espace interne du tube d'aiguille (34) contre le rétablissement de la forme enroulée d'une manière telle que le câble (35) est supporté par des parties de la surface interne du tube d'aiguille (34) qui sont espacées les unes des autres dans une direction de l'axe longitudinal du tube d'aiguille (34) ;
une partie fendue (34f) formée pour s'ouvrir de la surface intérieure à la surface extérieure du tube d'aiguille (34), la partie fendue (34f) présentant une paire de surfaces de paroi (34f-1, 34f-2) qui sont espacées l'une de l'autre, la partie fendue (34f) présentant une largeur d'ouverture supérieure à un diamètre externe du câble (35) ; et
un stylet (36) configuré pour pousser l'extrémité proximale du câble (35) chargé à l'intérieur du tube d'aiguille (34), dans lequel
le tube d'aiguille (34) comporte une partie de forme incurvée de tube d'aiguille (34A) formée au niveau d'un côté distal du tube d'aiguille (34), la partie de forme incurvée de tube d'aiguille (34A) présentant une élasticité telle que la partie de forme incurvée de tube d'aiguille (34A) est rétablie à une forme incurvée en l'absence d'une force externe,
le tube d'aiguille (34) présente une partie distale de tube d'aiguille comportant une pointe d'aiguille (34h) formée pour être aiguisée pour perforer un tissu à l'intérieur d'une cavité corporelle, la partie distale de tube d'aiguille étant formée au niveau d'un côté plus distal que la partie de forme incurvée de tube d'aiguille (34A),
une section de la surface interne en regard de la partie fendue (34f) supporte le câble (35) à l'intérieur du tube d'aiguille (34), d'une manière telle que le câble (35) est rétabli à la forme enroulée dans le plan d'observation ultrasonore (10A, 110A),
la partie fendue (34f) est formée au niveau de la partie distale de tube d'aiguille d'une manière telle que la partie fendue (34f) est en communication avec l'ouverture d'extrémité distale (34a) du tube d'aiguille (34), et s'ouvre vers un intérieur de la forme incurvée de la partie de forme incurvée de tube d'aiguille (34A), et
dans un état dans lequel le tube d'aiguille (34) fait saillie depuis le canal d'insertion (27) et l'extrémité distale du câble (35) est poussée depuis la partie fendue (34f) par le stylet (36), la paire de surfaces de paroi (34f-1, 34f-2) supporte le câble (35) d'une manière telle que le câble (35) est pris en sandwich entre les surfaces de paroi formant une paire (34f-1, 34f-2).

2. Aiguille de ponction (30) selon la revendication 1, dans laquelle
chacune des parties de la surface intérieure du tube d'aiguille (34) supportant le câble (35) est positionnée sur un plan prescrit.

3. Aiguille de ponction (30) selon la revendication 1, dans laquelle
dans un état dans lequel l'extrémité distale du câble (35) est agencée à l'intérieur de la partie de forme incurvée de tube d'aiguille (34A) positionnée dans le canal d'insertion (27), à mesure que le canal d'insertion (27) est courbé par la partie flexible (2b), la partie de forme incurvée de tube d'aiguille (34A) suit une courbe du canal d'insertion (27), et le câble (35) reçoit une force depuis la surface interne du tube d'aiguille (34) dans la partie de forme incurvée de tube d'aiguille (34A), de sorte que le câble (35) tourne d'une manière telle que la forme enroulée du câble (35) suit la forme incurvée de la partie de forme incurvée de tube d'aiguille (34A), et l'extrémité distale du câble (35) est positionnée sur le plan d'observation ultrasonore (10A, 110A).

4. Aiguille de ponction (30) selon la revendication 1, dans laquelle
dans un état dans lequel le câble (35) est supporté par les parties de la surface intérieure du tube d'aiguille (34) qui sont espacées l'une de l'autre dans la direction de l'axe longitudinal du tube d'aiguille (34), les parties supportant le câble (35) sont positionnées sur un plan qui comprend la partie fendue (34f) et qui se situe le long d'un axe central du tube d'aiguille (34), et les parties maintiennent le câble (35) entre une extrémité proximale de la partie fendue (34f) et le stylet (36).

5. Aiguille de ponction (30) selon la revendication 2, dans laquelle
dans un état dans lequel l'extrémité distale du câble (35) est rétablie à la forme enroulée dans le plan d'observation ultrasonore (10A, 110A) et la paire de surfaces de paroi (34f-1, 34f-2) supporte le câble (35) d'une manière telle que le câble (35) est pris en sandwich entre les surfaces de paroi formant une paire (34f-1, 34f-2), l'extrémité proximale du câble (35) et une partie centrale entre l'extrémité distale et l'extrémité proximale du câble (35) sont positionnées sur le plan prescrit.

6. Aiguille de ponction (30) selon la revendication 2, dans laquelle
l'extrémité distale, l'extrémité proximale, et une partie centrale entre l'extrémité distale et l'extrémité proximale du câble (35) sont positionnées sur le plan prescrit.

7. Aiguille de ponction (30) selon la revendication 1, dans laquelle
l'extrémité distale du câble (35) est positionnée au niveau de la partie de forme incurvée de tube d'aiguille (34A) dans un état dans lequel le câble (35) est étiré et chargé dans le tube d'aiguille (34).

8. Aiguille de ponction (30) selon la revendication 1, dans laquelle
une partie centrale du câble (35) est positionnée au niveau de la partie de forme incurvée de tube d'aiguille (34A) dans un état dans lequel le câble (35) est étiré et chargé dans le tube d'aiguille (34).

9. Aiguille de ponction (30) selon la revendication 1, dans laquelle
une extrémité proximale de la partie fendue (34f) est positionnée au niveau de la partie de forme incurvée de tube d'aiguille (34A).
